(19) Europäisches Patentamt / European Patent Office / Office européen des brevets

(11) **EP 3 419 549 B1**

(12) # EUROPÄISCHE PATENTSCHRIFT

(45) Veröffentlichungstag und Bekanntmachung des Hinweises auf die Patenterteilung:
**03.03.2021   Patentblatt 2021/09**

(21) Anmeldenummer: **17712018.5**

(22) Anmeldetag: **22.02.2017**

(51) Int Cl.:
*A61B 90/50* $^{(2016.01)}$          *A61B 17/00* $^{(2006.01)}$
*A61B 90/00* $^{(2016.01)}$          *A61B 46/10* $^{(2016.01)}$

(86) Internationale Anmeldenummer:
**PCT/EP2017/000247**

(87) Internationale Veröffentlichungsnummer:
**WO 2017/144172 (31.08.2017 Gazette 2017/35)**

(54) **HALTERUNG FÜR EIN INSTRUMENT**

MOUNTING FOR AN INSTRUMENT

SUPPORT D'INSTRUMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.02.2016   DE 202016001102 U**

(43) Veröffentlichungstag der Anmeldung:
**02.01.2019   Patentblatt 2019/01**

(73) Patentinhaber: **Isys Medizintechnik GmbH**
**6370 Kitzbühel (AT)**

(72) Erfinder: **VOGELE, Michael**
**86830 Schwabmünchen (DE)**

(74) Vertreter: **K&P Patentanwaltsgesellschaft mbH**
**Niederlassung Mindelheim**
**Fuggerstr. 2**
**87719 Mindelheim (DE)**

(56) Entgegenhaltungen:
**EP-A1- 2 937 056          EP-A2- 0 792 620**
**WO-A1-2012/077672          WO-A2-2006/072026**
**DE-U1-202013 105 792          US-A1- 2005 010 197**
**US-B2- 7 182 731          US-B2- 7 674 270**

EP 3 419 549 B1

**Beschreibung**

Die Erfindung betrifft eine Halterung für ein Instrument, insbesondere für ein medizintechnisches Instrument. Eine derartige Halterung ist aus WO 2012/0777672 bekannt, welche die oberbegrifflichen Merkmale des Anspruchs 1 zeigt.

Ein Beispiel eines Instruments ist ein "kleiner" Roboter, mit dem minimal-invasive Eingriffe am Patienten möglich sind. Dabei wird das Instrument durch Drehung von gelenkig angeordneten Armen der Halterung vorpositioniert und nach Arretierung der Arme somit die räumliche Position des Instruments fixiert. Bevorzugt wird hierbei eine sterile Hülle verwendet, um mindestens Teile der Halterung und des Instruments steril abzuschirmen. Aus der US 7,674,270 B2 ist eine Halterung bekannt, die vollständig von einer Hülle umgeben ist.

Insbesondere für kleinere, leichte Instrumente ist eine Halterung bekannt, die sich mittels Klemmschrauben und damit verbundenen Handrädern fixieren lässt. Solche Arten der Halterung sind z. B. auf den Produktseiten der Fa. Fisso (www.fisso.com) beschrieben. Diese Halterungen haben die Vorteile, dass sie kompakt und preiswert in der Herstellung sind.

Der Durchmesser der Handräder ist jedoch auf die Handgröße beschränkt, so dass bei größeren erforderlichen Drehmomenten das Festziehen für den Operator oder das Operationspersonal eine erhebliche Kraftanstrengung bedeuten kann. Weiterhin kann vom Operator kaum erkannt werden, ob die Halterung genügend stark fixiert ist. Dies kann bei zu schwacher Fixierung zum unbeabsichtigten Lösen der Halterung führen.

Im US-Patent 6,079,681 wird eine Halterung beschrieben, die ein medizinisches Instrument hält. Die Positionierung erfolgt dabei über das Verstellen der verschiedenen Bauteile des Halters und als Fixiermechanismus werden Schrauben und Handräder verwendet, die jedoch nur eine relativ ungenaue Haltekraft ermöglichen.

Eine weitere Möglichkeit, einstellbare Halterungen in einer gewünschten Position zu fixieren, ist der Einsatz einer Flüssigkeit in Art einer hydraulischen Klemmung. Dabei wird durch die Fluidzufuhr ein Bremsmechanismus aktiviert, die eine Bewegung der Halterung hemmt. Eine solche Lösung wird bereits im Patent DE 520270 A aus dem Jahr 1931 beschrieben. Dieser Lösungsansatz hat zwar den Vorteil, dass zur Fixierung im Prinzip nur ein Knopfdruck zur Zuführung des Fluids notwendig ist und dass durch geeignete Zuführung mehrere Gelenke gleichzeitig fixiert werden können. Nachteilig ist jedoch der komplexe Aufbau. Zudem sind bei einer solchen Lösung die Bedingungen zur Hygiene bzw. Sterilität wegen der nötigen Ventile und Schläuche kaum einzuhalten, insbesondere bei pneumatischer Ausführung.

Eine elektrische Lösung zur Fixierung wird beispielsweise in der DE 195 21 060 A1 gezeigt. Dabei wird die Bremswirkung in den Gelenken durch piezoaktive Aktoren bewirkt. Solche Halterungen mit elektronisch aktivierter Fixierung werden beispielsweise von der Firma SurgiTAIX unter dem Namen EndoTAIX angeboten. Nachteilig ist auch hier die komplizierte Leitungsführung und Installation sowie das benötigte Steuergerät.

Um eine sterile Operationsumgebung zu schaffen, wurden verschiedene Möglichkeiten vorgeschlagen, um den Roboter, die Halterung oder zumindest Teile davon abzudecken (vgl. US 6132 368 oder WO 2009/123925A1). Diese Vorschläge haben den Nachteil, dass die verwendeten sterilen Hüllen oftmals nicht formangepasst an die zu umgebenden Elemente sind, so dass diese platzfordernd sind und den Operator sogar behindern können. Es sind auch sterile Hüllen bekannt (z. B. aus der DE 10 2010 040 415 A1), deren Öffnungen sich bei Erwärmung schließen. Diese Anwendung lässt zwar einen sterilen Verschluss zu, aber die Anwendung von Wärme bringt einen weiteren Arbeitsvorgang mit sich, was gerade bei Notfall-Operationen unerwünscht ist.

Daher liegt der Erfindung die Aufgabe zugrunde, eine einfach und sicher zu fixierende Halterung, insbesondere für ein chirurgisches Instrument zu schaffen. Sowohl die Positionierung als auch die Fixierung soll dabei durch einfache mechanische Mittel erreicht werden, so dass auch schwere Instrumente oder Roboter für minimal-invasive Eingriffe am Patienten sicher eingesetzt werden können.

Diese Aufgabe wird durch eine Halterung mit den Merkmalen des Anspruches 1 gelöst. Vorteilhafte Ausgestaltungen sind Gegenstand der Unteransprüche.

Die vorgeschlagene Halterung bezieht sich insbesondere auf chirurgische Instrumente. Im Folgenden wird oftmals 'Roboter' zur Bezeichnung des Instruments verwendet, da die Verwendung eines solchen "Klein-Roboters" zur Feinjustierung ein bevorzugtes Ausführungsbeispiel ist. Die Anwendung wird nachfolgend an Hand der Zeichnungen beschrieben. Hierbei zeigen:

Fig. 1 eine Halterung eines Roboters, mit dem minimal-invasive Eingriffe am Kopf eines Patienten durchgeführt werden; und
Fig. 2 eine Detaildarstellung des mittigen, fixierbaren Gelenks der Halterung.

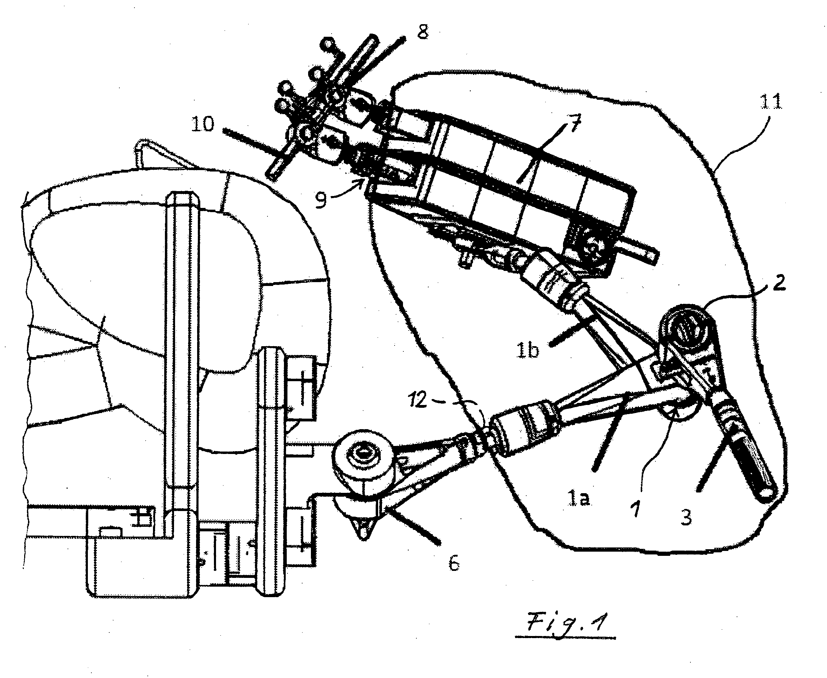

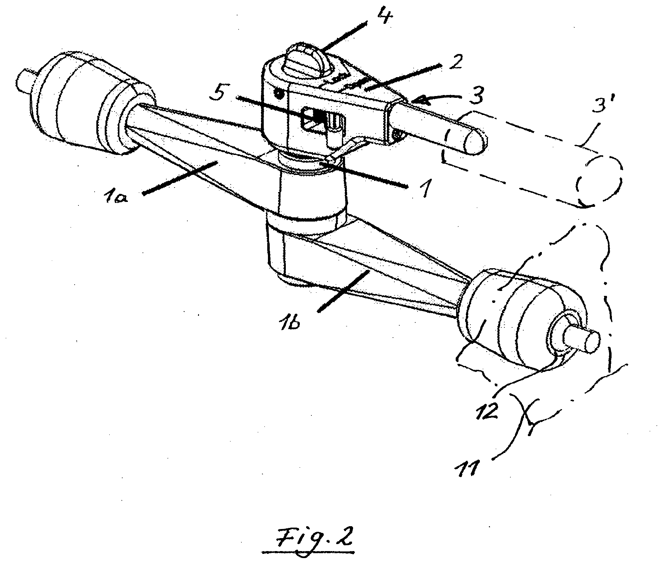

Die Halterung nach Fig. 1 weist zwei Arme 1a, 1b auf, die mit einem Drehkörper (mittiges Gelenk) 1 verbunden sind und gegeneinander in einer Dimension (Dreh-Freiheitsgrad) beweglich sind, d.h. der Winkel zwischen den Armen 1a, 1b kann beliebig gewählt werden. Meist spannen diese Arme 1a und 1b einen Winkel von ca. 90° auf, da der "untere" Arm 1a am OP-Tisch befestigt ist, oder wie hier an einer stabilen Verankerung 6 und quasi als Verankerungspfosten dient, während der "obere" Arm 1b in Art eines Kran-Auslegers auf die Operationsstelle hin ausgerichtet ist. Dies gilt auch für Wirbelsäulen-Eingriffe, wobei der Arm 1a die Körperhöhe von ca. 25 cm (Brustkorb-Bereich) überbrücken muss und der Arm 1b von der Seite her nahe zum Rückgrat reichen soll. Es sind auch andere Winkeleinstellungen, je nach Anwendungsfall der Halterung möglich (vgl. z. B. 180° Ausrichtung in Fig. 2).

Das Gelenk bzw. allgemein der Drehkörper 1, der beide Arme 1a, 1b verbindet, ist derart gestaltet, dass er sich über eine Kupplung 2 durch die Anwendung eines Drehmomentschlüssels in Form einer Ratsche 3 in einer Position festziehen lässt. Die Kupplung 2 ist hier (z. B. über einen Vierkant) auf den Gelenkförmigen Drehkörper 1 aufgesteckt. Die Kupplung kann auch in das verbreiterte Ende der Ratsche 3 integriert sein. Die Arme 1a und 1b sind bevorzugt aus Leichtmetall hergestellt und können je nach Anwendung verschiedene Längen aufweisen. Ein an der Kupplung 2 angebrachter Schalter 5, vgl. Fig. 2, beispielsweise ein Kipphebel, wird beim Feststellen der Position der Arme 1a, 1b zueinander in eine 'Lock'-Position eingestellt. In dieser 'Lock'-Position ist die Winkelstellung der Arme 1a, 1b arretiert, um mit einem bestimmten Drehmoment über die Ratsche das Gelenk am Drehkörper 1 mit definierter Haltekraft zu fixieren, die Arretierung jedoch nicht zu lösen. Dadurch wird verhindert, dass sich die eingestellte Position der beiden Arme 1a, 1b zueinander durch Bewegungen des Operateurs bzw. Operationspersonals die Fixierung unabsichtlich gelöst wird. Durch Umlegen des Schalters 5 in eine andere Schalterstellung kann die Arretierung wieder gelöst werden. Eine unabsichtliche Veränderung der Position/Schaltstellung des Kipphebel-Schalters 5 kann weiterhin durch Verwendung eines Riegelmechanismus verhindert werden.

Die Ratsche 3 kann durch Betätigung einer hier knaufförmigen Arretiervorrichtung 4 von der Kupplung 2 entfernt werden. Die Ratsche 3 ist bevorzugt mit einer Drehmomentbegrenzung versehen und kann auch einen aufsteckbaren Akku 3' aufweisen, wie in Fig. 2 in Strichlinien angedeutet. Die Drehmomentbegrenzung verhindert ein zu starkes Anziehen des Gelenks bzw. Drehkörpers 1 und damit Schäden an der Halterung. Weiterhin gewährleistet eine geeignete Wahl der Drehmomentbegrenzung eine definierte Haltekraft und somit stabile Fixierung der Arme 1a, 1b zueinander. Ratschen bzw. Drehmomentschlüssel 3 kürzerer Bauweise erlauben eine Fixierung des Gelenks auch bei beschränktem verfügbarem Raum, während längere Ratschen 3 eine bequeme Fixierung des Gelenks bis zur Drehmomentbegrenzung bei geringerer Kraftanstrengung des Operateurs erlauben. Je nach dem Wert der Drehmomentbegrenzung, dessen grobe Berechnung unten erläutert wird, können verschiedene Drehmomentschlüssel mit fester Drehmomentbegrenzung oder eine Ratsche 3 mit einstellbarer Drehmomentbegrenzung verwendet werden. Der Begriff "Ratsche" bezeichnet hierbei, wie im Werkzeug- oder Mechanikerbereich üblich, einen Schraubenschlüssel, der ohne Umstecken oder Rundum-Bewegung durch Vor- und Zurückschwenken (von je z. B. 60°) zum Festdrehen von Gewinden u. dgl. eingesetzt wird. Dieser begrenzte Schwenkbereich ist vorteilhaft, da das Platzangebot ohnehin beschränkt ist. Dabei gibt es auch Ratschen, die durch Drehen entlang der Hauptachse am Handgriff (vgl. die Ausrichtung des Akku 3') einsetzbar sind, also ohne größere Bewegung ein Gewinde oder Schraubverbindung (hier zum Fixieren des Gelenks am Drehkörper 3) "anziehen" können.

Das tischseitige Ende des Arms 1a wird an einer geeigneten Stelle durch eine Verankerung 6 fixiert. Eine bevorzugte Stelle ist eine seitliche Schiene am Operationstisch, nahe der Operationsstelle am Patienten, wobei sich die horizontale Position der Verankerung 6 durch Verschieben verändern lässt. Proximal zum Patienten ist ein chirurgisches Instrument 7 am Arm 1b angebracht; in einem bevorzugten Anwendungsbeispiel ein für minimal-invasive Eingriffe geeigneter Roboter oder mindestens ein Stellantrieb. Mit dem Roboter ist eine fernsteuerbare Feinpositionierung des für den operativen Eingriffs vorgesehenen Teils 10 (z. B. Injektionsnadel) möglich, das durch Haltestäbe 9 und einer Zieleinrichtung 8 am Roboter befestigt wird.

Im Allgemeinen ist eine sterile Hülle 11, beispielsweise aus einem Plastikmaterial vorgesehen, um die gesamte Anordnung oder zumindest Teile davon zu umgeben. In Fig. 1 wird praktisch die gesamte Halterung mit Roboter 7 von der sterilen Hülle 11 umschlossen, um somit eine Infektion des Patienten an der Operationsstelle durch Verunreinigungen an der Halterung mit Roboter zu verhindern. Dabei kann auch der Roboter bzw. Stellantrieb von einem Akku angetrieben werden, um den Verkabelungsaufwand zu minimieren. Geeignete Anschlüsse für die Ratsche 3 und auch für den Arm 1a am OP-Tisch ermöglichen eine Anbringung und einen Abbau der Halterung zusammen mit dem Roboter, ohne dass die sterile Hülle entfernt oder beschädigt wird. Durch die Verwendung eines luftdichten Anschlusses für die Ratsche 3, z. B. über in die Hülle 11 eingepasste O-Ringe, ergibt sich die Anwendungsmöglichkeit, den Drehmomentschlüssel auch außerhalb der sterilen Hülle 11 zu betätigen. Auch Teile der Haltestäbe 9 und/oder der Armee 1a, 1b können so luftdicht von der sterilen Folie 11 umschlossen werden, indem O-Ringe 12 an geeigneten Stellen in der Hülle 11 vorgesehen sind. Die Folie kann so von den Haltestäben 9 oder den Armen 1a, 1b durch die O-Ringe 12 hindurch durchstoßen werden, wie dies in Fig. 2 für den Arm 1b gezeigt ist (hierbei ist nur ein kleiner Teilbereich der Hülle 11 in Strichpunktlinien angedeutet).

Nachdem die Halterung am Operationstisch fixiert ist, wird die Position der beiden Arme 1a, 1b relativ zueinander eingestellt und mit der Ratsche 3 mit Drehmomentbegrenzung fixiert. Die Halterung wird durch den Schalter 5 in ihrer Verriegelungsstellung arretiert und die Ratsche 3 kann, falls gewünscht, durch Lösen der Arretiervorrichtung 4 entfernt werden. Dabei kann die innerhalb der Hülle 11 verbliebene Ratsche 3 auch von außerhalb der Hülle 11 leicht bewegt ("angezogen") werden, was bei der angesprochenen Benutzung von Handrädern durch die dazu notwendige Rundum-Rotationsbewegung viel schwieriger ist. Nach dieser Vor-Positionierung wird durch den Roboter die chirurgische Behandlung am Patienten, der vorher am Operationstisch fixiert worden ist, vorgenommen. Bei einer Injektion kann beispielsweise die Instrumentenfixierung wie in WO 2014/063828 A1 gezeigt, Anwendung finden und ein Nadelvorschub, wie in der DE 10 2013 004 062 A1 beschrieben, vorgenommen werden. Die Fixierung des Patienten kann beispielsweise wie in WO 2012/143142 A1 am Operationstisch vorgenommen werden. Mit dieser vorgestellten Halterung ist es möglich, durch einen einfachen Aufbau mit mechanischen Mitteln ein Instrument zu positionieren und sicher zu fixieren. Der kompakte Aufbau erlaubt es auch, die Teile der Halterung luftdicht mit einer sterilen Hülle zu umgeben.

**Anwendungsbeispiel:**

1. Wahl des einzustellenden Drehmoments

Um die Halterung wie beschrieben anwenden zu können, wird zunächst eine Abschätzung des notwendigen eingestellten Drehmoments der Ratsche 3 vorgenommen. Der geeignete Wert ergibt sich aus dem Drehmoment, das die Halterung des Roboters und der Roboter selbst auf das Gelenk (Drehkörper 1) ausübt.

Nimmt man für den Arm 1b eine Masse von 0,5 kg, eine Länge von 0,4 m und für den Roboter eine Masse von 3 kg an, so ist das resultierende Drehmoment

$$M = 9,81 \cdot 0,4 \cdot (½ \cdot 0,5 + 3) \text{ Nm} = 12,8 \text{ Nm}$$

Dieses am Drehkörper 1 anzulegende Drehmoment ist für die gegebenen Werte maximal, wenn eine horizontale Position des Arms 1b angenommen wird. Ratschen mit einstellbaren Drehmomenten in dieser Größenordnung sind käuflich erhältlich (vgl. Internetseite http://www.drehmomentschluessel-tests.org), wobei sich auch Ratschen 3 aus der Zahnmedizin (z. B. zur Fixierung von Kiefer-Implantaten) gut eignen.

Um ein Loslösen der Fixierung am mittigen Gelenk bzw. Drehkörper 1 zu verhindern, z. B. durch eine unabsichtliche Bewegung des Operateurs gegen die Halterung, sollte der gewählte Wert der Drehmomentbegrenzung etwas höher als der geschätzte Wert sein (z. B. mit einem Sicherheitsaufschlag von 50%).

2. Gewichtsbegrenzung

Als realistische Gewichte der Gesamtheit der zu tragenden Elemente werden obige Werte für die Masse des Roboters von 3 kg und der Masse des Arms 1b von 0,5 kg verwendet. Um die Gesamtmasse zu erhalten, müssen noch die Masse des Arms 1a von wiederum 500 g, die Masse des Drehkörpers 1 von z. B. 500 g, die Masse der Kupplung 2 von 500 g und die Masse der Ratsche 3 von 500 g, sollte diese nicht entfernt werden, addiert werden. Die Gewichte der Injektionsnadel 10, der Haltestäbe 9, der Zieleinrichtung 8 und der Hülle 11 sind vernachlässigbar. Man erhält ein Gesamtgewicht von 5,5 kg, was eine sichere Befestigung am OP-Tisch ermöglicht, der z. B. für einen Grenzwert von 8 kg ausgelegt ist.

**Patentansprüche**

1. Halterung mit einem Drehkörper (1) und zwei daran angelenkten Armen (1a, 1b) zur Einstellung von deren Winkel zueinander und Halten eines Instruments (7), wobei der eingestellte Winkel durch eine am Drehkörper (1) über eine Kupplung (2) angebrachte Ratsche (3) feststellbar ist und an der Kupplung (2) ein Schalter (5) vorgesehen ist, der die Winkelposition der Arme (1a, 1b) in einer Schalterstellung zueinander verriegelt,
**dadurch gekennzeichnet, dass**
die Kupplung (2) und mindestens ein Stellantrieb (7) in einer sterilen Hülle (11) angeordnet sind, welche die Arme (1a, 1b) luftdicht umhüllt, wobei wenigstens einer der Arme (1a, 1b) und/oder Haltestäbe (9) wenigstens teilweise einen runden Querschnitt besitzen und die sterile Hülle (11) an Durchtrittsöffnungen O-Ringe (12) aufweist, an denen einer der Arme (1a, 1b) und/oder die Haltestäbe (9) passgenau und luftdicht abschließen.

2. Halterung nach Anspruch 1, **dadurch gekennzeichnet, dass**
die Ratsche (3) eine feste oder einstellbare Drehmomentbegrenzung aufweist und vorzugsweise batterie- oder akkubetrieben (3') ist.

3. Halterung nach Anspruch 1 oder 2, **dadurch gekennzeichnet, dass**
am Arm (1b) distal vom Drehkörper (1) mindestens ein abnehmbar befestigter Stellantrieb (7) angebracht ist, der eine Zieleinrichtung (8) trägt und der nach Einstellung und Arretierung der Arme (1a, 1b) vorpositionierte und fixierte Stellantrieb (7) das chirurgische Instrument (10) über zwei Haltestäbe (9) feinpositioniert.

**4.** Halterung nach einem der Ansprüche 1 bis 3, **dadurch gekennzeichnet, dass** am Arm (1a) distal vom Drehkörper (1) eine Verankerung (6) vorgesehen ist.

**5.** Halterung nach einem der Ansprüche 1 bis 4, **dadurch gekennzeichnet, dass** eine Arretiervorrichtung (4) zur Verbindung der Ratsche (3) mit der Kupplung (2) vorgesehen ist.

**Claims**

**1.** Holder comprising a rotating body (1) and two arms (1a, 1b) articulated thereon for setting the angle thereof with respect to one another and holding an instrument (7), wherein the set angle is locked by means of a ratchet (3) which is attached to the rotating body (1) via a coupling (2), and a switch (5) being provided on the coupling (2) which locks the angular position of the arms (1a, 1b) in a switch position relative to one another, **characterized in that** the coupling (2) and at least one actuator (7) are arranged in a sterile sheath (11) which sheathes the arms (1a, 1b) in an airtight manner, at least one of the arms (1a, 1b) and/or holding rods (9) at least partially having a round cross section and the sterile sheath (11) having O-rings (12) at passage openings, on which O-rings one of the arms (1a, 1b) and/or the holding rods (9) closes in a precisely fitting and airtight manner.

**2.** Holder according to claim 1, **characterized in that** the ratchet (3) has a fixed or adjustable torque limitation and is preferably battery operated or rechargeable-battery operated (3').

**3.** Holder according to either claim 1 or 2, **characterized in that** at least one removably fastened actuator (7) is attached to the arm (1b) so as to be distal from the rotating body (1), which actuator supports a targeting device (8), and the actuator (7), which is prepositioned and fixed after setting and locking of the arms (1a, 1b), finely positions the surgical instrument (10) via two holding rods (9).

**4.** Holder according to any of claims 1 to 3, **characterized in that** an anchor (6) is provided on the arm (1a) so as to be distal from the rotating body (1).

**5.** Holder according to any of claims 1 to 4, **characterized in that** a locking device (4) is provided for connecting the ratchet (3) to the coupling (2).

**Revendications**

**1.** Support comportant un corps rotatif (1) et deux bras (1a, 1b) articulés sur celui-ci et destinés à régler l'angle formé entre les bras et à supporter un instrument (7), l'angle réglé pouvant être immobilisé par un cliquet (3) monté sur le corps rotatif (1) par l'intermédiaire d'un élément d'accouplement (2), et un commutateur (5) étant prévu sur l'élément d'accouplement (2), lequel commutateur verrouille la position angulaire des bras (1a, 1b) l'un par rapport à l'autre dans une position de commutation, **caractérisé en ce que** l'élément d'accouplement (2) et au moins un actionneur (7) sont disposés dans une enveloppe stérile (11), laquelle enveloppe les bras (1a, 1b) de manière étanche à l'air, au moins l'un des bras (1a, 1b) et/ou l'une des tiges de support (9) présentant au moins partiellement une section transversale ronde et l'enveloppe stérile (11) présentant des joints toriques (12) au niveau d'ouvertures de passage, joints toriques sur lesquels l'un des bras (1a, 1 b) et/ou les tiges de support (9) se ferment en ajustement précis et de façon étanche à l'air.

**2.** Support selon la revendication 1, **caractérisé en ce que** le cliquet (3) présente un limiteur de couple fixe ou réglable et est de préférence alimenté par batterie ou par accumulateur (3').

**3.** Support selon la revendication 1 ou 2, **caractérisé en ce qu'**au moins un actionneur (7) fixé de façon amovible est monté sur le bras (1b) de manière distale par rapport au corps rotatif (1), lequel actionneur porte un dispositif de visée (8) et l'actionneur pré-positionné et fixé (7) positionnant avec précision l'instrument chirurgical (10) par l'intermédiaire de deux tiges de support (9), après le réglage et le blocage des bras (1a, 1b).

**4.** Support selon l'une des revendications 1 à 3, **caractérisé en ce qu'**un ancrage (6) est prévu sur le bras (1a) de manière distale par rapport au corps rotatif (1).

5. Support selon l'une des revendications 1 à 4, **caractérisé en ce qu'**un système de blocage (4) est prévu pour relier le cliquet (3) à l'élément d'accouplement (2).

Fig.1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 20120777672 A
- US 7674270 B2
- US 6079681 A
- DE 520270 A
- DE 19521060 A1
- US 6132368 A
- WO 2009123925 A1
- DE 102010040415 A1
- WO 2014063828 A1
- DE 102013004062 A1
- WO 2012143142 A1